(19) Europäisches Patentamt
European Patent Office
Office européen des brevets

(11) **EP 1 084 112 B1**

(12) ## EUROPEAN PATENT SPECIFICATION

(45) Date of publication and mention
of the grant of the patent:
**03.03.2004 Bulletin 2004/10**

(51) Int Cl.[7]: **C07D 251/60**, C07D 251/62

(21) Application number: **99919709.8**

(86) International application number:
**PCT/NL1999/000264**

(22) Date of filing: **03.05.1999**

(87) International publication number:
**WO 1999/058508 (18.11.1999 Gazette 1999/46)**

(54) **METHOD FOR PREPARING MELAMINE**

VERFAHREN ZUR HERSTELLUNG VON MELAMIN

PROCEDE DE PREPARATION DE MELAMINE

(84) Designated Contracting States:
**AT DE ES FR GB IT NL SE**

(30) Priority: **12.05.1998 US 85065 P**

(43) Date of publication of application:
**21.03.2001 Bulletin 2001/12**

(73) Proprietor: **DSM IP Assets B.V.**
**6411 TE Heerlen (NL)**

(72) Inventor: **WANG, Yin**
**Bellingham, WA 98226 (US)**

(56) References cited:
**EP-A- 0 747 366**    **NL-A- 8 105 027**
**US-A- 3 116 294**    **US-A- 3 308 123**
**US-A- 3 386 999**

Remarks:
The file contains technical information submitted after the application was filed and not included in this specification

**Description**

The invention relates to a method for preparing melamine from urea via a high-pressure process in which solid melamine is obtained by transferring the reactor product comprising liquid melamine, $CO_2$ and $NH_3$ to a gas/liquid separator and subsequently transferring the melamine melt coming from the separator to a vessel where the melamine melt is cooled with a cooling medium.

Such a method is described, inter alia, in EP-A-747366. This describes a high-pressure process for preparing melamine from urea. In particular, EP-A-747366 describes how urea is pyrolysed in a reactor at a pressure of from 10.34 to 24.13 MPa and a temperature of from 354 to 454°C to produce a reactor product. The reactor product obtained contains liquid melamine, $CO_2$ and $NH_3$ and is transferred under pressure as a mixed stream to a gas/liquid separator. In this gas/liquid separator, which is kept at virtually the same pressure and temperature as the said reactor, the said reactor product is separated into a gaseous stream and a liquid stream. The gaseous stream contains $CO_2$ and $NH_3$, waste gases and also melamine vapour. The liquid stream mainly comprises liquid melamine. The gaseous product is transferred to a scrubber unit, while the liquid melamine is transferred to a product-cooling unit. In the scrubber unit, the said $CO_2$ and $NH_3$ waste gases, which contain melamine vapour, are scrubbed with molten urea, at virtually the same pressure and temperature as the pressure and temperature of the reactor, to preheat the urea and to cool the said waste gases to a temperature of 177-232°C and to remove the melamine present from the waste gases. Then the preheated molten urea, which contains the said melamine, is fed to the reactor. In the product-cooling unit, the liquid melamine is cooled with a liquid cooling medium, which forms a gas at the temperature of the liquid melamine in the product cooler, to produce a solid melamine product without scrubbing or further purification. In EP-A-747366 liquid ammonia is preferably used as the liquid cooling medium, the pressure in the product-cooling unit being above 4.14 MPa.

The purity of the melamine end product, according to EP-A-747366, is above 98.5 wt%, but it is not easy to achieve this level continuously on a constant level and on a commercial scale. This is a drawback, in particular when melamine is used in melamine-formaldehyde resins which are used in laminates and/or coatings. The impurities in the melamine end product, particularly when prepared on a commercial scale, consists primarly of melam, melem and oxygen containing impurities like ammelide, ammeline and urediomelamine.

The object of the present invention is to obtain an improved process for preparing melamine from urea, in which melamine is obtained as a dry powder having a high degree of purity. More in particular, the object of the present invention is to obtain an improved high-pressure process for preparing melamine from urea, in which melamine is obtained directly from the liquid melamine melt as a dry powder having a high degree of constant purity via cooling.

This object is achieved in that the gas/liquid separation is conducted by addition of $5.10^{-4}$ - $2.10^{-2}$ mole of water per mole of melamine. The water added to the gas/liquid separator is preferably chosen to be lower than $10^{-2}$ mole per mole melamine and higher than $10^{-3}$ mole per mole melamine. This ratio is a ratio of the feed rates to the separator.

It has surprisingly been found that with the process according to the invention the amounts of oxygen-containing impurities in the melamine end product can be kept constant.

The advantage of the method according to the present invention is that a powdered melamine is obtained of a purity which is constant and above 98.5 wt%, which is sufficient for the melamine thus obtained to be used in virtually any melamine application. At the same time it is possible to obtain melamine powder having very good colour characteristics.

The use of water in order to prevent the formation of oxygen-containing impurities has never been distinguished in the prior art. US-A-3116294 describes for example that, in order to obtain melamine with a purity higher than 99 wt%, raw melamine with a purity of 95% has to be heated in the presence of $NH_3$ alone explicitly in absence of water.

US-A-3,386,999 discloses the use of water vapour in a low-pressure catalytic melamine synthesis process. The water vapour should be added to the gaseous stream leaving the reaction phase, prior to the quench. The purpose of the water vapour is to hydrolyse cyanic acid and other melamine precursors.

NL-A-8105027 discloses that the method of US-A-3,386,999 is rather slow and will not lead to complete conversion; rather, a solid catalyst should be used when conversion of compounds such as cyanic acid into ammonia and carbon dioxide is desired.

US-A-3,308,123 discloses the use of a water spray in the separator/quencher of a high-pressure melamine process, the separator/quencher being at a temperature below 200°C. The process is designed such that contact between the water and the melamine is avoided as much as possible.

It is therefor the more surprisingly that such good results are obtained with the process according to the invention in which the gas/liquid separation is performed in the presence of water. The water may be added to the gas/liquid separator, preferably via a pump.

The amount of water added to the gas/liquid separator in the process according to the invention is between $5.10^{-4}$ and $2.10^{-2}$ mole of water per mole of melamine. The water added to the gas/liquid separator is preferably chosen to be between $10^{-3}$ and $10^{-2}$ mole of water per mole of melamine. It is hypothesized that water reacts with isocyanic acid into ammonia an carbondioxide which will leave the melamine melt. So the amount of isocyanic acid in the melamine melt will be reduced by the reaction with water. The advantage is a less and constant ammelide content in the melamine which ammelide is believed to be produced from the isocyanic acid.

The preparation of melamine preferably starts from urea as the raw material in the form of a melt. $NH_3$ and $CO_2$ are by-products during the preparation of melamine, which proceeds according to the following reaction equation:

$$6\ CO(NH_2)_2 \rightarrow C_3N_6H_6 + 6\ NH_3 + 3\ CO_2$$

The preparation can be carried out at high pressure, preferably between 5 and 25 MPa, without the presence of a catalyst. The reaction temperature ranges between 325 and 450°C and is preferably between 350 and 425°C. The by-products $NH_3$ and $CO_2$ are usually recycled to an adjoining urea plant.

The above-mentioned objective of the invention is achieved by employing an apparatus suitable for the preparation of melamine from urea. An apparatus suitable for the present invention may comprise a scrubber unit, a reactor, a gas/liquid separator, optionally a post-reactor and a cooling vessel.

In an embodiment of the invention, melamine is prepared from urea in an apparatus comprising a scrubber unit, a melamine reactor, a gas/liquid separator and a cooling vessel. Urea melt from a urea plant is fed to a scrubber unit at a pressure of from 5 to 25 MPa, preferably from 8 to 20 MPa, and at a temperature above the melting point of urea. This scrubber unit may be provided with a cooling jacket in order to ensure additional cooling within the scrubber. The scrubber unit may also be provided with internal cooling bodies. In the scrubber unit the liquid urea comes into contact with the reaction gases from the melamine reactor or from a separate gas/liquid separator downstream of the reactor. The pressure and temperature in the separate gas/liquid separator are virtually identical to the temperature and pressure in the melamine reactor. The reaction gases mainly consist of $CO_2$ and $NH_3$ and also comprise an amount of melamine vapour. The molten urea scrubs the melamine vapour from the waste gas and carries this melamine along back to the reactor. In the scrubbing process, the waste gases are cooled from the temperature of the reactor, i.e. from 350 to 425°C, to from 170 to 240°C, the urea being heated to from 170 to 240°C. The waste gases are removed from the top of the scrubber unit and, for example, recycled to a urea plant, where they are used as raw materials for the urea production.

The preheated urea is drawn off from the scrubber unit, together with the melamine scrubbed out, and supplied, for example via a high-pressure pump, to the reactor which has a pressure of from 5 to 25 MPa and preferably of from 8 to 20 MPa. Alternatively, the transfer of the urea melt to the melamine reactor may be effected by gravity, by the scrubber unit being positioned above the reactor.

In the reactor, the molten urea is heated to a temperature of from 325 to 450°C, preferably of from approximately 350 to 425°C, at a pressure as reported above, under which conditions the urea is converted into melamine, $CO_2$ and $NH_3$. A certain amount of ammonia can be metered into the reactor, for example in the form of a liquid or hot vapour. The ammonia supplied may serve, for example, to prevent the formation of condensation products of melamine such as melam, melem and melon, or to promote mixing in the reactor. The amount of ammonia supplied to the reactor is from 0 to 10 mol per mole of urea; preferably from 0 to 5 mol of ammonia is used and in particular from 0 to 2 mol of ammonia per mole of urea.

The $CO_2$ and $NH_3$ produced in the reaction as well as the additionally supplied ammonia collect in the separation section and are separated in the gaseous state from the liquid melamine.

To the gas/liquid separator downstream of the reactor is added $5.10^{-4}$ - $2.10^{-2}$ mole of water per mole of melamine. The water added to the gas/liquid separator is preferably chosen to be lower than $10^{-2}$ mole per mole melamine and higher than $10^{-3}$ mole per mole melamine.

To the gas/liquid separator downstream of the reactor, it may be advantageous for ammonia to be metered into this separator. The amount of ammonia in this case is 0.01-10 mol of ammonia per mole of melamine, preferably 0.1-5 mol. This has the advantage that the carbon dioxide is rapidly separated off, thus preventing the formation of oxygen-containing impurities. It is preferred that ammonia <u>and</u> water are present in the gas/liquid separator in order to prevent the formation of oxygen-containing impurities and to avoid the formation of melem, melam and melon.

The gas mixture formed after gas/liquid separation is passed to the scrubber unit in order to remove melamine vapour and preheat the urea melt.

The liquid melamine having a temperature between the melting point of melamine and 450°C is drawn off (from the reactor or) from the gas/liquid separator downstream of the reactor and, is rapidly cooled and depressurized using a liquid medium that is vapor at the conditions of the cooling unit, preferably ammonia.

**Claims**

1. Method for preparing melamine from urea via a high-pressure process in which solid melamine is obtained by transferring the reactor product comprising liquid melamine, $CO_2$ and $NH_3$ to a gas/liquid separator and subsequently transferring the melamine melt coming from the separator to a vessel where the melamine melt is cooled, **characterized in that**, the gas/liquid separation is conducted by addition of $5.10^{-4}$ - $2.10^{-2}$ mole of water per mole of melamine.

2. Method according to claim 1, **characterised in that** the cooling of the melamine melt is done by means of an evaporating cooling medium.

3. Method according to claim 1 or 2, **characterized in that**, $10^{-3}$ - $10^{-2}$ mole of water per mole of melamine is added.

4. Method according to any one of claims 1-3, **characterized in that**, the gas-liquid separation is conducted by addition of 0.01-10 mole of ammonia per mole of melamine.

5. Method according to claim 4, **characterized in that**, the gas-liquid separation is conducted by addition of 0.1-5 mole of ammonia per mole of melamine.

## Patentansprüche

1. Verfahren zur Herstellung von Melamin aus Harnstoff über ein Hochdruckverfahren, worin festes Melamin durch Überführen des Reaktorproduktes, umfassend flüssiges Melamin, $CO_2$ und $NH_3$, zu einem Gas-Flüssig-Separator und nachfolgendes Überführen der Melaminschmelze, welche aus dem Separator kommt, zu einem Gefäß, worin die Melaminschmelze abgekühlt wird, erhalten wird, **dadurch gekennzeichnet, daß** die Gas-Flüssig-Trennung durch Zugabe von $5^{.}10^{-4}$ - $2^{.}10^{-2}$ Mol Wasser pro Mol Melamin durchgeführt wird.

2. Verfahren nach Anspruch 1, **dadurch gekennzeichnet, daß** das Abkühlen der Melaminschmelze mittels eines Verdampfungskühlmittels durchgeführt wird.

3. Verfahren nach Anspruch 1 oder 2, **dadurch gekennzeichnet, daß** $10^{-3}$ - $10^{-2}$ Mol Wasser pro Mol Melamin zugegeben wird.

4. Verfahren nach einem der Ansprüche 1 bis 3, **dadurch gekennzeichnet, daß** die Gas-Flüssig-Trennung durch Zugabe von 0.01-10 Mol Ammoniak pro Mol Melamin durchgeführt wird.

5. Verfahren nach Anspruch 4, **dadurch gekennzeichnet, daß** die Gas-Flüssig-Trennung durch Zugabe von 0,1-5 Mol Ammoniak pro Mol Melamin durchgeführt wird.

## Revendications

1. Procédé de préparation de mélamine à partir d'urée au moyen d'un processus haute pression dans lequel on obtient de la mélamine solide en transférant le produit du réacteur comprenant de la mélamine liquide, du $CO_2$ et du $NH_3$ dans un séparateur gaz/liquide puis en transférant la mélamine en fusion issue du séparateur dans un récipient, dans lequel on refroidit la mélamine en fusion, **caractérisé en ce que** la séparation gaz/liquide est conduite par addition de $5.10^{-4}$ à $2.10^{-2}$ mole d'eau par mole de mélamine.

2. Procédé selon la revendication 1, **caractérisé en ce qu'**on conduit le refroidissement de la mélamine en fusion au moyen d'un agent de refroidissement par évaporation.

3. Procédé selon la revendication 1 ou 2, **caractérisé en ce qu'**on ajoute $10^{-3}$ à $10^{-2}$ mole d'eau par mole de mélamine.

4. Procédé selon l'une quelconque des revendications 1 à 3, **caractérisé en ce qu'**on conduit la séparation gaz-liquide en ajoutant 0,01 à 10 moles d'ammoniac par mole de mélamine.

5. Procédé selon la revendication 4, **caractérisé en ce qu'**on conduit la séparation gaz-liquide en ajoutant 0,1 à 5 moles d'ammoniac par mole de mélamine.